# EUROPEAN PATENT SPECIFICATION

(11) **EP 3 798 614 B1**
(45) Date of publication and mention of the grant of the patent: **20.11.2024**
(21) Application number: 20150390.1
(22) Date of filing: 06.01.2020
(51) Int. Cl.: G01N 21/64, G01N 21/01, G01N 21/77, A61B 5/00, G01N 21/84

(54) **MODULAR MULTIPLEX ANALYSIS DEVICES AND PLATFORMS**
MODULARE MULTIPLEX-ANALYSE-VORRICHTUNGEN UND -PLATTFORMEN
DISPOSITIFS ET PLATEFORMES D'ANALYSE MULTIPLEX MODULAIRE

(30) Priority: 30.09.2019 US 201962908477 P
(43) Date of publication of application: 31.03.2021
(73) Proprietor: IAssay, Inc., San Diego CA 92129 (US)
(72) Inventor: ADELMAN, Lonnie, San Diego, CA 92129 (US)
(74) Representative: Clark, Jane Anne

(56) References cited:
- EP-A1- 1 403 795
- WO-A1-2018/152573
- WO-A2-2018/148471
- US-A1- 2003 119 202
- ONUR MUDANYALI ET AL: "Integrated rapid-diagnostic-test reader platform on a cellphone", LAB ON A CHIP, vol. 12, no. 15, 2012, pages 2678 - 2686, XP055058669, ISSN: 1473-0197, DOI: 10.1039/c2lc40235a

## Description

### BACKGROUND

Point of care testing is an important way for healthcare providers to effectively and efficiently provide care to individuals.

Point of care testing allows for testing and monitoring of individuals in a plurality of settings including a hospital, clinic, remote reference lab testing facility, doctor's office, home of individual being tested, or anywhere in which an individual may be tested or monitored.

A paper by Onur Mudanyali et al entitled "Integrated rapid-diagnostic-test reader platform on a cellphone", published in Lab on a Chip describes a cellphone-based rapid-diagnostic-test (RDT) reader platform that can work with various lateral flow immune-chromotographic assays and similar tests to sense the presence of a target analyte in a sample. The digital RDT reader mechanically attaches to an existing camera unit of a cellphone and various types of RDT can be inserted to be imaged in reflectional transmission mode under light emitting diode-based illumination. Captured raw images of these tests are then digitally processed through a smart application running on the cellphone for validation of the RDT, as well as for automated reading of its diagnostic result.

EP 1403795 A1 describes an information communication system including a storage medium in which target information is stored in advance, an information processor for inputting the target information from the storage medium, and an analyzer for inputting the target information from the information processor.

WO 2018/148471 A2 describes optics, device and system for assaying including imaging

WO 2018/152573 A1 describes a diagnostic system including a test device for performing diagnostic analysis of a biological sample from a human or animal body, the test device having an assay and a reader to analyse the sample and determine one or more biological conditions and to generate a result data signal containing information on the determination of the one or more biological conditions. The system also comprises a base device to releasably receive the test device, receive the result data signal from the test device, and display results of testing based on the received result data signal.

US 2003/0119202 A1 describes an optical reflectance kit including a reading device and membrane test strip is disclosed for conducting a lateral flow assay. Assays may be conducted on bodily fluids to detect with high sensitivity the presence of certain hormones, glucose, or other bodily fluids. Membrane test strips may receive a test fluid or test sample containing an analyte to be detected.

### SUMMARY OF THE INVENTION

The present invention is set out in the appended claims. Point of care testing, or the carrying out of laboratory tests outside of a traditional laboratory setting, has grown and expanded as a practice over the past 20 years driven at least in part by improvements in technology as well as a marked increase in at-home patient care.

However, while its use has increased greatly over the past 20 years, it is known among healthcare providers that point of care testing has long been and remains highly inefficient for both the point of care testing provider as well as the patient undergoing point of care testing. The inefficiency of point of care testing is due to, at least, the: (1) physical cumbersomeness of the point of care testing technology as well as the (2) cumbersomeness of the use of point of care technology.

Typically, a point of care testing provider, using traditional technology, must utilize multiple point of care readers each reader having its own specific assay containers which are each typically configured to perform a single (or multiple) laboratory test. That is, a single reader is typically designed to operate with specific sample containers and testing components that fit with and operate with that specific reader. Thereby, currently, each test performed at the bedside or at a walk-in site requires multiple readers each utilizing multiple assay containers (i.e. each assay container from a particular manufacturer requires its own reader from the same manufacturer).

The multiple readers currently used in point of care testing are physically cumbersome to carry from test site to test site, and also take up critical bench space at a physician's office lab or a pharmacy because the multiple readers cumulatively have a significant weight and cumulatively take up a fair amount of space. As a result, a point of care testing provider typically is unable to add additional tests, either ordered by the physician or requested by the patient while they are at the site because they do not typically carry all of the testing tools with them to a site. To add these additional tests typically means the point of care testing provider must leave and return to the site with the necessary testing tools - an obvious source of inefficiency at the point of care.

The multiple readers currently used with traditional point of care testing technology are cumbersome to use, and because the healthcare provider typically create a confusion for the healthcare provider in terms of correctly matching up each individual reader with its own module after a sample has been taken. As a result, traditional point of care testing is cumbersome to carry and difficult to use.

Described herein is a hand-held modular point of care testing device, system, and platform configured to test individuals and simultaneously and automatically collect data from tests performed on individuals in a point of care testing setting. A hand-held modular point of care testing device includes a housing or frame that includes a user interface for device operation, including inputting operator selections, displaying test data, communicating with connected devices and/or the Cloud and displaying data from connected devices and/or the Cloud. The hand-held modular point of care testing device addresses a long felt but unmet need with respect to traditional point of care testing technology for reducing the cumbersomeness and inefficiency in existing technology in that the instant innovative device, for example: (1) Efficiently collects and transmits data from multiple different types of input sources and (2) Provides multiplex analysis for multiple different tests and sample types.

### Delivery of Efficient Point of Care Testing

A hand-held modular point of care testing device, system, or platform as described herein is efficient in numerous ways including in how it collects data from multiple data sources and transmits data to a network or networks and/or server or servers that are accessible to healthcare providers and laboratory personnel.

The instant innovation comprises a hand-held modular point of care testing device that in some embodiments includes ports or openings configured to receive one or more assay modules and, in some embodiments, such ports or openings include connections that provide for interoperability with different assay modules from different manufacturers. Interoperability with different types of assay modules reduces the amount of equipment that a point of care testing provider needs to have on hand at a point of care testing site since the instant hand-held modular point of care testing device functions as a single universal receiver for different assay modules. In some embodiments, samples may be received directly by the device (or within a container that is received directly by the device). In some embodiments, a test module wirelessly transmit data a hand-held modular point of care testing device described herein or the assay module itself functions as a self-contained hand-held modular point of care testing device.

In some embodiments, a hand-held modular device includes a receiver for wirelessly receiving data and is further configured to wirelessly receive data from one or more modules, other devices, or networks (including a cloud-based network, i.e. the Cloud). A hand-held modular point of care testing device, in some embodiments, includes a transmitter for transmitting data either wirelessly or through a wired connection from one or more modules, other devices, or networks (including a cloud-based network). In some embodiments, a hand-held modular point of care testing device includes a data reader including, for example, an RFID reader and/or a camera or other imaging modality. For example, in some embodiments, a device comprises a lightproof chamber with an aperture fitted with a lens and a shutter through which the image of an object is projected onto a surface for recording (as on a photosensitive film or an electronic sensor) or for translation into electrical impulses (as for television broadcast). In some embodiments, a device comprises a detector that, for example, detects a pattern of magnetic fields, and the location of the fields. For example, in some embodiments, a detector comprises a radio frequency detection as in the detection in a closed loop.

In general, a hand-held modular point of care testing device further includes a processor and memory which in some embodiments includes software configured to cause the processor to carry out certain functions. In some embodiments, a hand-held modular point of care testing device includes a power source such as a battery.

A modular hand-held device as described herein is modular in that the device is configured to operate with and/or receive data from multiple different sources which includes multiple different types of modules, wherein each module may be specifically configured to carry out at least one assay or test. The modular hand-held device described herein is configured to operably couple with one or more modules so that data is transmitted from the module to the hand-held modular point of care testing device (such as to a memory and/or a processor of a hand-held modular point of care testing device). In general, a modular hand-held device is configured to provide interoperability between different assay modules including those made by different manufacturers. In this way, a modular hand-held device is configured to function as a universal reader for different types of assays.

In some embodiments, a hand-held device as described herein is a component in a platform. A platform as described herein includes a graphic user interface portal on a device for use at a point of care site as well as one or more other user portals that are configured to communicate (i.e. transmit data) with one another. In some embodiments, a healthcare provider portal is a software application on a computing device that is configured to receive data from a device as described herein used for point of care testing. In some embodiments, a healthcare provider portal is configured to allow a healthcare provider to transmit instructions to device used for point of care testing, for example, a lab order request for a particular type of lab. In some embodiments, a platform as described herein includes a user portal which is a software application running on a computing device that is configured to receive and display data transmitted to the user by a data source within the platform. In some embodiments, a platform allows different devices and device types to communicate with each other. In this way, a modular hand-held device as part of a platform functions to more efficiently transmit and receive data (e.g. healthcare data) to and from multiple devices and to and from multiple users, something that traditional point of care devices are not configured to do.

### Multiplex Analysis

A modular hand-held device as described herein is configured, in some embodiments, to perform multiplex sample analysis. A multiplex sample analysis includes a configuration wherein different sample types are analyzed using a single device and/or module. A multiplex sample analysis includes a configuration wherein a single type of sample is analyzed using different assays and/or tests. A multiplex sample analysis includes a configuration wherein one or more sample types are assayed or tested and results from said assays or tests are combined with other data or physical parameters associated with said one or more samples.

Described herein is a modular hand-held point of care testing system comprising: a testing module configured to receive a biologic sample and containing one or more reagents that mix with the biologic sample when it is received by the testing module; a housing configured to receive said testing module; a user interface positioned on a surface of said housing including a digital display; a processor configured to receive data from the testing module and user interface; and a universal interoperability coupler configured to operably couple said testing module with said processor. In some embodiments, said testing module includes an imaging modality such as, for example, a camera configured to capture an image of the sample and one or more reagents when the sample and the one or more reagents are mixed together. In some embodiments, an imaging modality or detector is independent of the testing module. For example, a one or more cameras and/or detectors, in some embodiments are positioned over every module port. In some embodiments, said processor includes software configured to cause the processor to evaluate said image. In some embodiments, said testing module is configured for multiplex testing by receiving at least two samples at once that are separated within the testing module. In some embodiments, wherein the at least two samples are positioned on a test strip. In some embodiments, the imaging modality determines a physical boundary of each of the at least two samples that are separated. In some embodiments, the system comprises a fixed area aperture where an average is taken of anything within the aperture. For example, if a test line on the strip is narrower than the aperture, and average is taken in parts of the bare strip or plastic from the cassette with the capture line. In some embodiments, an operator is able to fine turn the position of the entire aperture, or move the edges of the aperture to get a more perfect fit compared to the position found mathematically. In some embodiments, said one or more reagents change color when mixed with a sample. In some embodiments, the color change is identified by the camera. In some embodiments, a system comprises a radioactivity detector. In some embodiments, the digital display includes a touchscreen controller configured to receive input from a user that is transmitted to the processor. The universal interoperability coupler comprises an adaptor for reflective reading reagent test cartridges of different dimensions internal adjustment bars with external handles that allow for the user to preset the external handles to positions that hold the cartridge in place. In some embodiments, further comprising a wireless transceiver configured to wirelessly transmit and receive data. In some embodiments, the transceiver is configured to transmit the data to a server. In some embodiments, the system comprises a non-transitory computer readable medium including software configured to cause the processor to display a user-portal on the digital display, the user-portal configured to provide a communication link with another user portal on a different device. In some embodiments, the transceiver operates on one or more transmission technologies selected from 3G communication protocols, 4G communication protocols, 5G communication protocols, GSM standards, CDMA protocols, IEEE 802.11 standards, Bluetooth protocols, satellite communications, visible light communications, infrared communications, and near field communications. In some embodiments, the system comprises a non-transitory computer readable medium including software configured to provide communication protocols to allow communication with other computing devices through one of a Bluetooth connection, a WiFi connection, hardwired USB (with or without adaptor dongles) and other types of RF connections. In some embodiments, the system further comprises a light pipe or a prism device lined-up with a camera embedded in the portable computing device. In some embodiments, the system is configured to operate with testing modules each manufactured by a different manufacturer. In some embodiments, the system is configured to receive at least two testing modules that each have a different format than the other. In some embodiments, the test module includes an RFID tag, and the housing includes an RFID tag reader. In some embodiments, the system further comprises a camera, wherein the camera comprises a lighting source, and a receiver configured to receive a fluorescent signal (including chemiluminescence). In some embodiments, the fluorescent signal is received from the test module. In some embodiments, the system comprises two or more cameras, wherein images formed by individual cameras are assembled to create a single image with a wider field of view. In some embodiments, the system further comprises a shroud. In some embodiments, the test module is labeled with a bar or QR code. In some embodiments, the system is configured to receive at least two test modules each having a different shape than the other. In some embodiments, the system comprises a vital sign detector configured to sense one or more vital signs of an individual. In some embodiments, the system comprises a luminescence recorder adapted to record luminescence associated with the biologic sample. In some embodiments, the luminescence recorder is selected from a camera, a fluorescent light recorder, a UV recorder, a diode/amplifier type receiver, or combinations thereof. In some embodiments, the luminescence recorder is a built-in camera of a portable computing device. In some embodiments, the test module is configured to carry out at least one of a Sodium assay, Potassium assay, Chloride assay, BUN/Urea assay, Glucose assay, Hemoglobin assay, Hematocrit assay, Ionized Calcium assay, PO2 assay, pH assay, PCO2 assay, Creatinine assay, Lactate assay, Celite ACT assay, Prothrombin Time PT/INR assay, Kaolin ACT assay, Cardiac Troponin I/cTnI assay, Total Carbon Dioxide/TC02 assay, Creatine Kinase MB/CK-MB assay, B-Type Natriuretic Peptide/BNP assay, Sepsis assay, an immunodiagnostic assay, a DNA sequencing assay, a bioluminescent assay, a cell cytometry assay, a lateral flow assay, and an HbAlc assays. In some embodiments, the immunodiagnostic assay is an enzyme-linked immunosorbent assays (ELISA). In some embodiments, the DNA sequencing assay is based on DNA sequencing chips or 2^{nd} gen, 3^{rd} gen sequencing systems that provide optical, magnetic, or electrical signaling (or any other detectable biochemical signal) and wherein the test module comprises an interface with the DNA sequencing chip or the 2^{nd} gen, 3^{rd} gen sequencing systems. In some embodiments, the test module is configured to carry out a lateral flow assay based on comparison of capture line intensity, line color, or a combination thereof to one or more capture lines. In some embodiments, the assay device comprises an optical device disposed for imaging the lateral flow assay, and a display device for visualizing the image of the lateral flow assay.

Described herein is a method for performing point of care testing comprising: receiving a testing module, configured to receive a biologic sample and containing one or more reagents that mix with the biologic sample when it is received by the testing module, within a port of a hand-held testing device; wherein a universal interoperability coupler within the port is configured to operably couple said testing module so that data is transferred from the testing module to a processor of the hand-held testing device. In some embodiments, said testing module includes a camera configured to take image an image of the sample and one or more reagents when the sample and the one or more reagents are mixed together. In some embodiments, said processor includes software configured to cause the processor to evaluate said image. In some embodiments, said testing module is configured for multiplex testing by receiving at least two samples at once that are separated within the testing module. In some embodiments, the at least two samples are positioned on a test strip. In some embodiments, the camera determines a physical boundary of each of the at least two samples that are separated. In some embodiments, said one or more reagents change color (including chemiluminescence) when mixed with a sample. In some embodiments, the color change is identified by the camera. In some embodiments, the digital display includes a touchscreen controller configured to receive input from a user that is transmitted to the processor.

The universal interoperability coupler comprises an adaptor for reflective reading reagent test cartridges of different dimensions internal adjustment bars with external handles that allow for the user to preset the external handles to positions that hold the cartridge in place. In some embodiments, the method further comprises transmitting a wireless signal from the hand-held testing device using a wireless transceiver configured to wirelessly transmit and receive data. In some embodiments, the transceiver is configured to transmit the data to a server (including a hardware and cloud based server). In some embodiments, the method comprises using software configured to cause the processor to display a user-portal on the digital display, wherein the user-portal configured to provide a communication link with another user portal on a different device. In some embodiments, the transceiver operates on one or more transmission technologies selected from 3G communication protocols, 4G communication protocols, 5G protocols, GSM standards, CDMA protocols, IEEE 802.11 standards, Bluetooth protocols, satellite communications, visible light communications, infrared communications, and near field communications. In some embodiments, the method comprises using software configured to provide communication protocols to allow communication with other computing devices through one of a Bluetooth connection, a WiFi connection. In some embodiments, the hand-held device includes a light pipe or a prism device lined-up with a camera embedded in the portable computing device. In some embodiments, the hand-held testing device is configured to operate with testing modules each manufactured by a different manufacturer. In some embodiments, the hand-held testing device is configured to receive at least two testing modules that each have a different format than the other. In some embodiments, wherein the test module includes an RF tag, and the housing includes an RF tag reader. In some embodiments, the hand-held testing device comprises a camera, wherein the camera comprises a lighting source, and a receiver configured to receive a fluorescent signal. In some embodiments, a capture device is separate from a lighting source. In some embodiments, a light source excites labels, and they can be different wavelengths.

In some embodiments, the fluorescent signal is received from the test module. In some embodiments, the hand-held testing device comprises two or more cameras, wherein images formed by individual cameras are assembled to create a single image with a wider field of view. In some embodiments, the hand-held device includes a shroud that, in some embodiments, shields ambient noise such as, for example, light from a light source or imaging device (e.g. a camera). In some embodiments, the test module is labeled with a bar or QR code. In some embodiments, the hand-held testing device is configured to receive at least two test modules each having a different shape than the other. In some embodiments, the hand-held testing device comprises a vital sign detector configured to sense one or more vital signs of an individual. In some embodiments, the hand-held testing device comprises a luminescence recorder adapted to record luminescence associated with the biologic sample. In some embodiments, the luminescence recorder is selected from a camera, a fluorescent light recorder, a UV recorder, a diode/amplifier type receiver, or combinations thereof. In some embodiments, the luminescence recorder is a built-in camera of a portable computing device. In some embodiments, the test module is configured to carry out at least one of a Sodium assay, Potassium assay, Chloride assay, BUN/Urea assay, Glucose assay, Hematocrit assay, Ionized Calcium assay, PO2 assay, pH assay, PCO2 assay, Creatinine assay, Lactate assay, Celite ACT assay, Prothrombin Time PT/INR assay, Kaolin ACT assay, Cardiac Troponin I/cTnI assay, Total Carbon Dioxide/TC02 assay, Creatine Kinase MB/CK-MB assay, B-Type Natriuretic Peptide/BNP assay, an immunodiagnostic assay, a DNA sequencing assay, a bioluminescent assay, a cell cytometry assay, a lateral flow assay, and an HbAlc assays. In some embodiments, the immunodiagnostic assay is an enzyme-linked immunosorbent assays (ELISA). In some embodiments, the DNA sequencing assay is based on DNA sequencing chips and wherein the test module comprises an interface with the DNA sequencing chip. In some embodiments, the test module is configured to carry out a lateral flow assay based on comparison of line intensity, line color, or a combination thereof to one or more capture lines. In some embodiments, test module is configured to carry out a lateral flow assay on a non-linear arrangement of multiple samples such as, for example, an array or matrix of samples. In some embodiments, the assay device comprises an optical device disposed for imaging the lateral flow assay, and a display device for visualizing the image of the lateral flow assay.

Described herein is a point of care testing device, comprising: a test module configured to receive a container that contains a biologic sample and generate an analysis result of said biologic sample and further comprising: a processor configured to receive data from the test module; and a transmitter configured to transmit data from said test module to a mobile computing device or a remote server; and a universal interoperability coupler configured to operably couple said test module with said container. In some embodiments, said test module includes a camera configured to take an image of the sample. In some embodiments, said processor includes software configured to cause the processor to evaluate said image. In some embodiments, said test module is configured for multiplex testing by receiving at least two samples at once that are separated within the test module. In some embodiments, the at least two samples are positioned on a test strip. In some embodiments, the camera determines a physical boundary of each of the at least two samples that are separated. In some embodiments, the universal interoperability coupler comprises an adaptor for reflective reading containers of different dimensions. The universal interoperability coupler comprises one or more internal adjustment bars with external handles that allow for the user to preset the external handles to positions that hold the cartridge in place. In some embodiments, the transmitter is configured to wirelessly transmit and receive data. In some embodiments, the transmitter operates on one or more transmission technologies selected from 3G communication protocols, 4G communication protocols, GSM standards, CDMA protocols, IEEE 802.11 standards, Bluetooth protocols, satellite communications, visible light communications, infrared communications, a hardwired connection, and near field communications. In some embodiments, said device comprises a non-transitory computer readable medium including software configured to provide communication protocols to allow communication with other computing devices through one of a Blue Tooth connection, a WiFi connection, and an RF connection. In some embodiments, the device is configured to operate with containers each manufactured by a different manufacturer. In some embodiments, the test module includes an RF tag. In some embodiments, the device further comprises a camera, wherein the camera comprises a lighting source, and a receiver configured to receive a fluorescent signal. In some embodiments, the fluorescent signal is received from the test module. In some embodiments, the device comprises two or more cameras, imaging modalities, or detection devices, wherein images formed by individual cameras or individual imaging modalities are assembled to create a single image with a wider field of view. In some embodiments, the device comprises a shroud or shield configured to shield from ambient noise. In some embodiments, the test module is labeled with a bar code. In some embodiments, the device is configured to receive at least two containers each having a different shape than the other. In some embodiments, a vital sign detector configured to sense one or more vital signs of an individual. In some embodiments, a luminescence recorder adapted to record luminescence associated with the biologic sample. In some embodiments, the luminescence recorder is selected from a camera, a fluorescent light recorder, a UV recorder, a diode/amplifier type receiver, or combinations thereof. In some embodiments, the luminescence recorder is a built-in camera of a portable computing device. In some embodiments, the test module is configured to carry out at least one of a Sodium assay, Potassium assay, Chloride assay, BUN/Urea assay, Glucose assay, Hematocrit assay, Ionized Calcium assay, PO2 assay, pH assay, PCO2 assay, Creatinine assay, Lactate assay, Celite ACT assay, Prothrombin Time PT/INR assay, Kaolin ACT assay, Cardiac Troponin I/cTnI assay, Total Carbon Dioxide/TC02 assay, Creatine Kinase MB/CK-MB assay, B-Type Natriuretic Peptide/BNP assay, an immunodiagnostic assay, a DNA sequencing assay, a bioluminescent assay, a cell cytometry assay, a lateral flow assay, and an HbAlc assays. In some embodiments, the immunodiagnostic assay is an enzyme-linked immunosorbent assays (ELISA). In some embodiments, the DNA sequencing assay is based on DNA sequencing chips and wherein the test module comprises an interface with the DNA sequencing chip. In some embodiments, the test module is configured to carry out a lateral flow assay based on comparison of line intensity, line color, or a combination thereof to one or more reference lines. In some embodiments, the assay device comprises an optical device disposed for imaging the lateral flow assay, and a display device for visualizing the image of the lateral flow assay.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG. 1** shows an exemplary embodiment of a modular hand-held point of care testing device.
**FIG. 2** shows an example of environment that can be employed to execute implementations of one or more embodiments of the platform.
**FIG. 3** shows an exemplary embodiment of a test module **300** as described herein configured as a lateral flow cartridge.
**FIG. 4** is a schematic representation of a device as described herein coupled with three test devices that are coupled to one another to form a multiplex testing device.

### DETAILED DESCRIPTION

Described herein is a hand-held point of care monitoring device, system, and platform configured to carry out tests and measurements including, but not limited to, biologic sample testing and physical parameter (e.g. vital sign) measurement.

### Definitions

As used herein, the term "individual" means any human or animal.

As used herein, the term "point of care" means any location wherein a hand-held testing device may be utilized and/or any location at which health care may be delivered to an individual. Non-limiting examples of a point of care includes a home of a patient, a health clinic or doctor's office, a mobile clinic or other field clinic site, a nursing home, a rehabilitation facility, or hospital.

As used herein the term "transmission" or "transmitter" includes any modality for transmitting a signal including both wired and wireless modalities.

As used herein, the term "modular" means comprising one or more components or parts. It should be further understood that one or more modular components or parts may be interchangeable with other components or parts. Typically, as used herein, an interchangeable component or part may be removed and added by a user of the modular item by, for example, a reversibly couple-able mechanism such as those well known in the art.

As used herein a "module" means an object or device that is a component of a modular system, device, or platform.

As used herein a "test module" means a module configured to carry out a test (or similar analytical function). A test module may be configured to receive a sample directly through an opening in the test module. In some embodiments, a test module may be configured to couple with (including wirelessly) a sample container such as, for example, a cartridge, test strip, or cuvette. In these embodiments, where a test module receives a container of a sample, the container may also comprise a testing device that includes electronics configured to analyze a sample within the container, and the test module is configured to receive data from the container. In some embodiments, a test module is configured to analyze a sample directly and, in some embodiments, a test module is configured to read sample analysis data from a container that it couples with (including wirelessly) or that it receives within it (i.e. the test module receives the container). A test module may be manufactured specifically to pair with the a modular hand-held point of care testing device described herein or as used herein a test module may be manufactured by one or more other manufacturers and not manufactured specifically to pair with the modular hand-held point of care testing device described herein.

As used herein a "container" means a receptacle for receiving and holding a sample and includes containers configured to carry out an analysis of a sample within said container (similar to a "test module") unless otherwise specified. For example, in some embodiments a test module is configured to receive a container. In some of these embodiments, a container is configured to hold a sample and, in some embodiments, a container is configured to carry out an analysis of a sample contained therein. Non-limiting examples of containers include cartridges and strips. In some embodiments, a container comprises a cartridge or strip comprising a reflective reading reagent.

As used herein a "device module" means a module configured to provide some specific functionality when coupled (including wirelessly) with or received by a device, system, or platform component.

### Devices

A modular hand-held point of care testing device as described herein is configured to function as a self-contained unit that runs a variety of laboratory tests on one or more specimens, and/or senses one or more data parameters, and provides a user interface which in some embodiments includes a digital touch screen controller display.

A modular hand-held point of care testing device, system, or platform is configured to carry out one or more laboratory tests on a sample or specimen taken from an individual and/or scan and/or receive data relating to a laboratory test. Non-limiting examples of different laboratory tests suitable to be carried out using embodiments of the device, system, and platform described herein include ABO Grouping (Blood Typing), Adrenocorticotropic Hormone level, Aldosterone level, Alpha 1 Antitrypsin level Alpha Fetoprotein level, Aluminum level, Amylase level, Antinuclear Antibody (ANA) Screen, Apolipoprotein A1 (Apo A1) level, Arsenic level, B12 level, Beta Carotene level, Beta HCG level, Bone-Specific Alkaline Phosphatase level, B-type natriuretic peptide level, Calcitonin, serum level, Calcium, Ionized level, Cancer Antigen 125 level, Cancer Antigen 15-3 level, Cancer antigen 27.29 level, Candida Antibodies level, Carbohydrate Antigen 19.9 level, Carcinoembryonic Antigen level Carnitine level, Catecholamine level, Celiac Disease Antibody Screen, Ceruloplasmin level, Chemistry Panel & Complete Blood Count (CBC), Chromium, plasma level, Chromogranin A level, Complement C3 level, Complement C4 level, Copper level, CoQ10 (Coenzyme Q10) level, Cortisol level, Cortisol 24 Hour level, Cortisol AM/PM level, Coxsackie Group B Antibodies, C-Peptide level, C-Reactive Protein level, Creatine Kinase level, C-Telopeptide, serum level, Cystatin C level, Cytokine Panel level, Cytomegalovirus (CMV) Antibodies, IgG, Cytomegalovirus (CMV) Antibodies, IgM, D-Dimer level, Dehydroepiandrosterone Sulfate level, Dihydrotestosterone level, Epstein Barr Virus, ESR, Estradiol level, Total Estrogen level, Estrone level, F2-Isoprostane level, Factor VIII Activity, Ferritin level, Fibrinogen level, Folate level, Fructosamine level, Galectin-3 level, Gamma Glutamyl Transferase level, Glutathione level, Gluten level, Helocobacter Pylori, IgG, Hemoglobin A1C level, Hepatitis B surface Antibody, Hepatitis C Virus Antibody, Homocysteine level, Human Herpes Virus Antibodies, Insulin-Like Growth Factor Binding Protein 3 (IGFBP-3), Intact N-Terminal Propeptide of Type 1 Procollagen (P1NP), Interleukin 6 (IL6), Interleukin 8 (IL-8), Interleukin 1beta (IL-1beta), Iodine level, Ionized Calcium level, Iron & Total Iron-Binding Capacity (TIBC), Lactate Dehydrogenase (LD) Isoenzymes, Leptin level, Lipase level, Lipoprotein (a) level, Lithium level, Magnesium level, Mercury level, Myeloperoxidase level, Osteocalcin level, Parathyroid Hormone level, Reticulocyte Count, Serotonin level, Sex Hormone Binding Globulin level, Transferrin level, Troponin I level, Tumor Necrosis Factor - Alpha level, Vitamin A level, Vitamin B1 level, Vitamin B12 level, Vitamin B6 level, Vitamin C level, Vitamin D level, Vitamin K1 level, Zinc level, Adrenocorticotropic Hormone level, Alkaline Phosphatase level, Aluminum level, Ammonia level, Antidiuretic Hormone level, Antinuclear Antibody, Arsenic level, B Type Naturetic Peptide level, Total Estrogen level, Progesterone level, Testosterone level, Prostate Specific Antigen level (PSA), C- Reactive Protein (High Sensitivity- Cardiac) level, Cadmium level, Calcium, Ionized (Serum) Test, Candida Antigen / Antibody Profile, Ceruloplasmin levels, Chlamydia Pneunomonia level, Complete Metabolic Panel, Copper level, Cortisol level, C-Peptide level, Dehydroepiandrosterone level, Dihydrotestosterone level, Epstein-Barr Virus level, Erythrocyte Sedimentation Rate, Estradiol level, Estriol level, Estrone level, Ferritin level, Folate level, Follicle-Stimulating Hormone level, Luteinizing Hormone level, Glucose-6-Phosphate Dehydrogenase level, Glutathione level, Growth Hormone level, Hemoglobin A1c level, Homocysteine level, IgA Immunoglobin level, IgE Immunoglobin level, Insulin level, Insulin Growth Factor (IGF-1), Iron level, Lactic Acid Dehydrogenase level, Lead level, Leptin level, Lipid level, Magnesium level, Manganese, Methylmalonic Acid level, Microalbumin level, Parathyroid Hormone level, Prolactin level, Prothrombin Time (PT), Partial Thromboplastin (PTT) Prothrombin Time INR, Reverse Triiodothyronine level, Selenium level, Sex Hormone-Binding Globulin level, T-3 Uptake, Testosterone Free and Total, Thyroglobulin, Thyroid Antibody level, Thyroid Stimulating Hormone level, Thyroxine (T4), Thyroxine Binding Globulin level, Tumor Necrosis Factor-Alpha, Uric Acid level, Total Cholesterol, HDL Cholesterol level, LDL Cholesterol level, Urine Specific Gravity (SG), Urine pH, Urine Protein level, Urine Glucose level, Urine Ketones, Urine Blood (hemoglobin) and Myoglobin, Urine Leukocyte Esterase, Urine Nitrite, Urine Bilirubin, Urobilinogen, and Fecal Occult Blood.

In some embodiments, a modular hand-held point of care testing device, system, or platform is further configured to sense a physiologic parameter by receiving data from one or more physiologic data sensors. Non-limiting examples of physiologic data sensed by embodiments of the device, system, and platform described herein include blood pressure, heart rate, spO2, temperature, weight, and galvanic skin response.

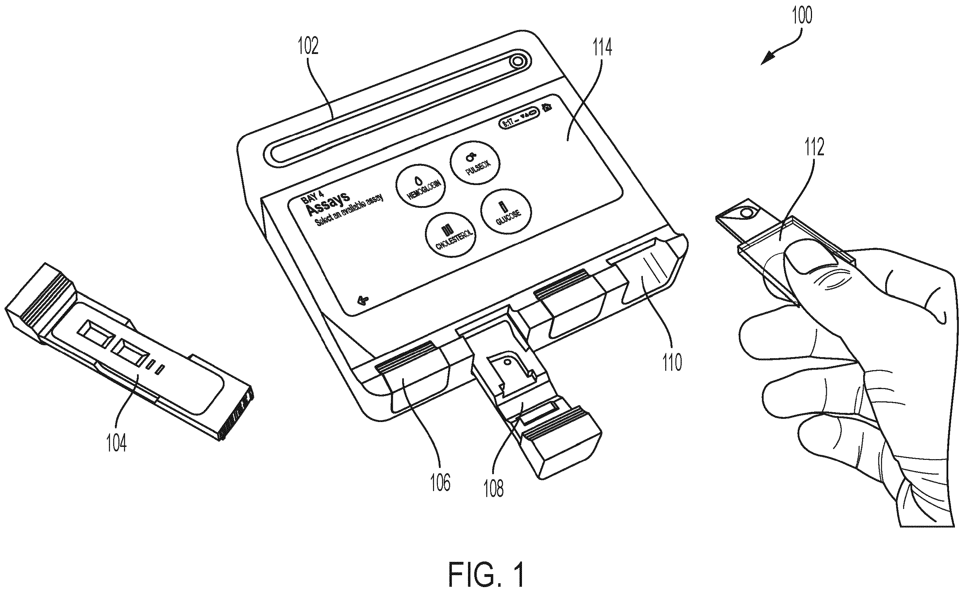

**FIG. 1** shows an exemplary embodiment of a modular hand-held point of care testing device (also referred to as "device") **102** and system (also referred to as "system") **100**. As shown, device **102** includes device module **106**, test module **108** which, in this embodiment, further includes removable cuvette **112,** module port **110**, and touch screen display **114**. As shown also, device **102** further comprises a housing (not labeled separately) that encases all of the components of device **102**. A housing of device **102** is generally sized and configured to be comfortably held in the hand of a typical user. In some embodiments, the housing is configured to rest on a surface when the device is in use. In some embodiments, a housing includes a strap configured to hold the device onto an operator's hand. In addition, device **102** is formed of generally lightweight components so that the total weight of device **102** will be one where the device will be comfortably held and carried when used.

In some embodiments, a test module **108**is configured to receive a sample containing container such as, for example, the cuvette shown in **FIG. 1**. In these embodiments, test module 108 further includes components that allow for the test module **108** to test or analyze the sample in the container that it has received. For example, a test module **108** may include an imager and/or detector for analyzing a sample within the cuvette. The imager of the test module **108** in this example may be configured, for example, to transmit a beam of energy (e.g. light, radiation) through the cuvette where it may be detected by a detector within the test module **108**. As described herein, a test container may itself be configured to analyze a sample within it and in these embodiments, the test module **108** is configured to receive data from the container.

In any of the embodiments described herein, a test module **108** may be configured to couple with a housing of a device **102** or may include transmitter components that are configured to transmit data wirelessly to a device **102**.

A test module **108** may be configured, in some embodiments, to transmit data a wirelessly to a network (e.g. The cloud), a remote server, or a computing device such as a smartphone. In some of these embodiments, a test module **108** is self-contained and does not couple or integrate with a device **100** but rather carries out an analysis or other data capture and transmits that data to a network, remote server, or computing device.

### Embodiments with Integrated Computing Devices

In an embodiment (not shown in **FIG. 1****)** a housing of a device **102** is configured to operably couple with a smartphone or other computing device so that one or more of the electronic components of the computing device including (e.g. the smartphone) are utilized as the electronic components of the device **102**. That is, in this embodiment, a device **102** is configured to couple with a computing device so that the computing device is operatively integrated with the device **102**. In some embodiments, the portable computing device is selected from a smart-phone or a tablet computer. In some embodiments, the computing device comprises a software module configured to provide step-by-step guidance for using the device **102.** In some embodiments, the computing device comprises a software module configured to collect, process and organize data the test module **108,** the device module **106**, or a combination thereof. In some embodiments, the computing device comprises software module configured to communicate data to a user, wherein the data is acquired from the test module **108**, the device module **106**, or a combination thereof. In some embodiments, the assay information is securely communicated to the user through a server. In some embodiments, the server is an Internet server or a local access network server. In some embodiments, the user is a patient, a doctor, or a nurse. Computing devices suitable for use in the present disclosure include, but are not limited to, mobile phones, mobile computing devices, smartphones, portable computers, tablet computers, and mobile computers.

### Embodiments Including Non-Modular Components

In some embodiments, a device **102** is as described above but further comprises one or more non-modular components configured to carry out one or more functions of the test module **108**, the device module **106**, or a combination thereof. In these embodiments, the non-modular component is affixed to or integrated with the device **102**. For example, a device **102** in some embodiments includes an opening or port configured to directly receive a sample or a container of a sample such as, for example, a cartridge, test strip, or cuvette. As described, said container, in some embodiments, includes components configured to analyze a sample within the container. For example, a device **102** includes certain electronic components affixed to or integrated with the device itself.

### Electronic Components

While not shown in **FIG. 1**, a device **102** further includes electronic components and circuitry including a microprocessor, a memory, and a transceiver (or, alternatively, separate transmitter and receiver). In some embodiments, the microprocessors are solo core, dual-core, quad-core, 8-core, 16-core, 32-core, 64-core microprocessors. The microprocessors may be graphic processors with one core or more than one core. In some cases, the microprocessors are microcontrollers and single processors. Alternatively, the microprocessor may be an electronic circuitry designed specifically to process the data described in this subject matter. In addition, a non-transitory memory includes software that is configured to cause the processor to carry out various processes described herein. In some embodiments, a device **102** comprises one or more cameras, detectors (e.g. electromagnetic field, radiation), or other imaging modalities. In some embodiments, a device **102** comprises a luminescence recorder (including chemiluminescence) adapted to record luminescence generated by the test module **108** as it tests a sample using a luminescence technique. In some embodiments, the luminescence recorder is selected from a camera, a fluorescent light recorder, a UV recorder, or combinations thereof. In some embodiments, the luminescence recorder is a built-in camera of a portable computing device. In some embodiment, the luminescence recorder comprises a light source and a light receiver, such as a pin diode/amplifier type receiver tuned to a specific wavelength.

In some embodiments, device **102** further comprises connectors that mate with the adaptor modules, USB hubs, cabling. printed circuit assemblies, and/or batteries.

### Housing

A housing of device **102** is configured to have at least one module port **110** which, in the embodiment shown, is an opening configured to receive a device module **106** or test module **108**. A module port **110** opening is sized to receive at least one device module **106** or test module **108** at one time, and, in some embodiments (not shown) a module port **110** opening is configured to receive two or more modules (either device module **106** or test module **108**) within the same port **110**. A port **110** may also contain coupling components that provide for removable coupling of the modules within the port **110.** Such removable coupling components are well known in the art and may, for example, include snap fit components, springs, and/or magnets. A port **110**, in some embodiments, includes coupler that provides for operable coupling between the device **102** and the device module **106** and/or test module **108**. That is, an operable coupler found within the port **110** is configured to connect the module within the port **110** to the device's electronic components including the device processor, transceiver, and memory. In some embodiments, an operable coupler within a port **110** comprises prong based coupler where conductive prongs on either a module or within the port **110** are positioned to mate with a conductive prong receiver on either the module or within the port **110** so that a conductive connection forms between the module within the port **110** and the port **110**. In these embodiments, there is additional circuitry connecting the conductive operable coupler within the port **110** to the electronics and circuitry of the device **102** so that electrical signals travel from the device **110** to the module within the port **110** and from the module within the port **110** to the electronic components and circuitry of the device **102**. It should be understood that other conductive mechanical coupling techniques are suitable or use with the device **102** including any other means of creating a detachable connection between a module within a port **110** and the port **110**.

In some embodiments, a device **102** includes at least one module that is permanently affixed to or integrated with the housing of the device **102**. That is, in this embodiment, not all modules are removable from the device **102**.

As described above, in some embodiments, a test module **108** is self-contained and does not couple further with a housing or otherwise physically integrate with a separate housing as part of a system. Rather, in these embodiments, a test module **108** is configured to either carry out an analysis on a sample within it or receive data related to a sample analysis from, for example, a container that has a sample within it and that is received by the test module **108**.

### Interoperability and Universal Interoperability Couplers

In general, the device **102** is configured to be interoperable with numerous different modules (either a device module **106** or test module **108**) including modules manufactured by different manufacturers and modules having different shapes, sizes, and components. The device **102** achieves this interoperability feature by including a universal interoperability coupler which may or may not comprise a mechanical mechanism for adjusting a size of a port **110**. For example, one or more walls of a port may be on an adjustable track so that the distances between walls enclosing a port can be adjusted making the overall size of enclosure of the port **110** adjustable. In these embodiments, this feature provides fitting of different sized test modules within an adjustable port **110** by making the port enclosure either larger or smaller in size. Similarly, a port **110** may include one or more adjustable bars within the port that run the length and/or width of the port **110** and when a bar is adjusted it effectively changes the width or length of a port **110.** For example, a port **110** may include an adjustable bar that runs across the width of port **110** (either entirely or partially) and when moved in a forward direction within the port **110** it results in a decrease in the length of the port **110** (i.e. decreases the volume of the opening within the port **110**). Likewise, a bar running the length of the port **110** (either entirely or partially) when moved in a lateral direction changes the width of the port **110** so that volume of the opening within the port **110** is changed.

In some embodiments, a test module **108** has an opening configured with an universal interoperability coupler feature as described herein such as the adjustable wall(s) or bar(s) that are configured to change the dimensions of an opening within a test module **108** that receives a sample, a container, and/or a testing device. As described, said container, in some embodiments, includes components configured to analyze a sample within the container. In this way, a test module **108** may be configured to receive containers having different dimensions or may be configured to receive multiple containers and/or samples at once.

Adjustable walls or bars, in some embodiments of the device **102** and test module **108**, include components that extend externally from the device **102** or test module **108** and are attached to one or more adjustable walls or bars so that a user can manually adjust the position of the adjustable wall or bar by moving the externally extending component. In some embodiments, an adjustable wall or bar within a device **102** or test module **108** is coupled to an actuator of the device **102** or test module **108** so that the adjustable wall or bar is moved automatically by the actuator, thereby resizing a port or opening in a device **102** or test module **108** so that the size of the opening is adjusted. In some embodiments that include an actuator, the actuator is coupled with a sensor that senses at least one dimension of a component (e.g. a sample container) to be received within the device **102** or test module **108** and the actuator is configured to adjust at least one wall or bar within the device **102** or test module **108** in order to accommodate the component within the device **102** or test module **108**.

In some embodiments, a device **102** is configured to receive multiple test modules **102** and/or samples at once and run a number of different tests in parallel by running tests using one or more test modules **102** and/or directly testing a sample that is received by the device **102**.

A universal operability coupler is in general a mechanism for creating a coupling between a module within a port **110** and a device **102**. In some embodiments, a prong coupling system (as described above) within the port **110** is configured to receive or mate with a corresponding prong receiver and/or prongs (depending on whether the prongs are within port **110** or positioned on a module). For example, a prong receiver within a port **110** may be configured to receive (i.e. couple with) multiple prong numbers and/or configurations. For further example, a prong receiver may have six openings which will allow it to mate with one prong, two prongs, three prongs, four prongs, five prongs, or six prongs. Or alternatively, there may be multiple prongs within a port **110** configured to mate with a prong receiver on a module and one or more of the prongs may be retractable so that the prongs within a port **110** can be adjusted to mate with a prong receiver having different numbers of openings. For further example, a port **110** may have a universal operable coupler within it comprising of six retractable prongs configured to mate with a prong receiver on a module having one hole (i.e. and five prongs are retracted), two holes (i.e. and four prongs are retracted), three holes (i.e. and three holes are retracted), four holes (i.e. and two prongs are retracted), five holes (i.e. and one prong is retracted) or six holes (i.e. and no prongs are retracted). Further, in the example of the prong coupling system, as explained, an electrical connection is created between the device **102** and the module when the prongs and prong receiver are mated (i.e. couple). This electrical connection provides for the one directional or bi-directional transfer of data between the device **102** and the module. The adjustability of the prong system in this embodiment, makes it a universal coupler so that no matter what the prong or prong receiver configuration on any module, the module can operatively couple with the device **102**.

In some embodiments , a device **102** includes wireless communication means configured to transfer data with a wireless communication means on a module as would a mechanical coupler (e.g. a prong based system). Non-limiting examples of wireless transmission technologies suitable for use as universal operable coupler in the device **102** include 3G transceivers, 4G transceivers, Bluetooth transceivers, visible light signal transceivers, infrared transceivers, RF transceivers, and near field transceivers.

In some embodiments, a port **110** has a configuration that is adjustable. An adjustable configuration of port **110** allows the port **110** to receive different modules each having a different configuration. That is, for example, a dimension of a port **110** such as, for example, a width of a port **110** may be adjustable in order to fit a plurality of modules each having a different width. Similarly, a dimension of port **110** may be adjusted in order to simultaneously fit a plurality of modules. It should be understood that a number of means are suitable for mechanically adjusting a configuration of a port **110** including, for example, using movable elements attached to the port **110** or, for example, removable fittings of different sizes. Device **102** includes four ports including port **110** but in other embodiments, a device **110** may have 1, 2, 3, 5, 6, 7, 8, 9, or 10 ports. It should be understood that any number of ports are suitable for use with device **110** while maintaining a hand-held configuration.

### Device and Test Modules

A device **102**, in some embodiments, is configured to receive a device module **106** and/or a test module **108** within any one of its ports. In general, a device module **106** provides a functionality and a test module **108** carries out or at least partially carries out a test on a sample taken from an individual. For example, a device module **106** may couple with the device **102** when in a port **110** and provide a reader, processor, or memory that is operably coupled with the electronics of device **102**. In some embodiments, one or more electronic components of device **102** are within one or more device modules **106** and device **102** is able to utilize whatever electronic components are in device module **106** while the device module **106** is in any port or ports of device **102**. A test module **108** in general comprises any module that carries out or assists in the carrying out of a test on a sample. When a test module **108** is inserted into one port of a device **102** (such as port **110**), the test module data is transferred to the device **102** through an operable coupling (such as, for example, a universally operable coupling). In some embodiments, a processor on board the device **102** is configured to control operation of a device module **106** and or a test module **108** that are received within one or more ports of the device **102**. It should be understood that the device module **106** and or a test module **108** are both configure to reversibly couple with the device **102** so that they can be interchanged by a user.

In some embodiments, a device module **106** can influence the way that the device functions too. A device module can process sample data more quickly than the processor in the device, then pass the processed answer to the device in order to more quickly process a sample or for other reasons. In some embodiments, a device module can simply hold a rechargeable battery that can back-power the device for long periods of time.

In some embodiments, at least two device modules have different shapes and the housing of device **102** comprises at least two slots with different shapes to releasably retain the at least two differently shaped modules. In some cases, two or more device modules are physically connected through an electric wire, a tube, or an optical fiber. In some embodiments, two or more functional modules are wirelessly coupled through a wireless communication.

In some embodiments, at least test modules are produced by different assay or test manufacturers. In some embodiments, the assays or tests of the test modules are produced in more than one assay format (e.g. lateral flow and electrochemical).

In some embodiments, the test module **108** is placed in a disposable holder. A test module **108**, in some embodiments, comprises a test cartridge. Test modules or test cartridges are physically held by a disposable holder in exactly the right position to be inserted as a group into mating portable frame (which bounds assay modules) or assay modules (which bounds test cartridges). Then the holder is removed and disposed of. The test cartridges held by the holder can be the same or different formats.

In some embodiments, the test module **108** includes an RF tag, the device **102** comprises an RF tag reader, and the test module **108** is coupled with the device **102** via the RF tag and RF tag reader. The RF tag, or so called RF ID tag, is inside the test module **108** so that the reader in the device **102** detects which type of **test module 108** has been inserted.

In some embodiments, a test module **108** is used for a specific family of tests, or for a single test. In some embodiments, the test module (e.g., lateral flow or electrochemical) has a tag on it so that when it is inserted into the assay module the portable frame and/or the assay module detects what test is to be run. Alternatively, test module **108** comprises an RF ID tag on the test module **108** that is read by the reader in the device **102** so that the system knows what test is being run.

In some embodiments, a device **102** and/or a test module **108** comprises a camera, other imaging modality, or a detector. Non-limiting examples of imaging modalities include X-ray imaging, ultrasound as well as video imaging, photometry, and microscopy. Non-limiting examples of detectors include luminescence detectors (including chemiluminescence) radiation detectors, RF detectors, electromagnetic field detectors, and barcode readers.

In some embodiments, a camera comprises (a) a lighting source, and (b) a receiver configured to receive a light signal (e.g., fluorescent signal) from the assay. In some embodiments, the light signal is received from the test module **108**. In some embodiments, the test module **108** further comprises two or more cameras or imaging modalities, wherein each of the cameras comprise a lighting source, and a receiver configured to receive a light signal. In some embodiments, the light signal is received from the test module **108**.

As described, in some embodiments, device **102** includes a camera or other imaging modality or detector that is positioned to be used to analyze a sample within, for example, a test module **108**. In some embodiments, a built in camera or other imaging modality or detector of a computing device coupled to device **102** is positioned to be used to analyze a sample within a test module **108**

In some embodiments, images formed by individual cameras or other imaging modalities or detectors are assembled to create a single image with a wider field of view. In some embodiments, images formed create an image captured with natural light. The embodiments are to artificially increase the field of view with two cameras. In some embodiments, the device **102** and/or test module **108** further comprises a shroud or shield configured to block ambient noise and/or interference.

In some embodiments, analyzing a signal comprises normalizing the signal. In some embodiments, test module **108** further includes a code. The code comprises one or more of the following: a bar code and a QR code. In some embodiments, the code is used to align or synchronize a code scanning process. The code can also be a series of marks (visible or not) that are detected as **108** is slid into **102**, or as 104 is slid into **108**, or read with 104 in place and **108** fully seated. The marks can help the system determine position, velocity, and acceleration, or to read some manufacturing code, etc. These marks can be like a bar code or QR code, or just a scale.

In some embodiments, at least two test modules have different shapes and the device **102** at least two ports with different shapes to releasably retain the at least two test modules. In some embodiments, at least two test modules have a same shape and the device **102** comprises at least two ports with a same shape to releasably retain the at least two test modules.

Tests and/or assays performed by a test module **108** generally include health care assays, veterinary assays, food product assays, and environmental assays.

In some embodiments, a test module **108** is configured to perform one or more of immunodiagnostic assays, DNA sequencing assays, bioluminescent assays, cell cytometry assays, and lateral flow assays.

Non-limiting examples of immunodiagnostic assays performed fully or in part by a test module **108** include enzyme-linked immunosorbent assays (ELISA). In some embodiments, the DNA sequencing assays are based on DNA sequencing chips and wherein the assay device comprises interface to the DNA sequencing chip. In some embodiments, the immunodiagnostic assays are enzyme-linked immunosorbent assays (ELISA). The ELISA (sometimes also called an EIA) is a sensitive, inexpensive assay technique involving the use of antibodies coupled with indicators (e.g. enzymes linked to dyes) to detect the presence of specific substances, such as enzymes, viruses, or bacteria. While there are several different types, basically ELISAs are created by coating a suitable plastic (the solid phase) with an antibody. To complete the reaction, a sample believed to contain the antigen of interest is added to the solid phase. Then a second antibody coupled with an enzyme is used followed by the addition of a color-forming substrate specific to the antibody. In some embodiments, the ELISA assay is based on comparison of color type, color intensity, or a combination thereof to one or more references. In some embodiments, the assay device comprises an optical device disposed for imaging or analyzing the ELISA assay, and optionally a display device for visualizing the image of the ELISA assay. In some embodiments, the immunodiagnostic assays are lateral flow assays. Lateral flow tests also known as Lateral Flow Immunochromatographic Assays are simple devices intended to detect the presence (or absence) of a target analyte in sample (matrix) without the need for specialized and costly equipment, though many lab based applications exist that are supported by reading equipment. Typically, these tests are used for medical diagnostics either for home testing, point of care testing, or laboratory use. A widely spread and well known application is the home pregnancy test. The lateral flow test is based on a series of capillary beds, such as pieces of porous paper or sintered polymer. Each of these elements has the capacity to transport fluid (e.g., urine) spontaneously. The first element (the sample pad) acts as a sponge and holds an excess of sample fluid. Once soaked, the fluid migrates to the second element (conjugate pad) in which the manufacturer has stored the so-called conjugate, a dried format of bio-active particles (see below) in a salt-sugar matrix that contains everything to guarantee an optimized chemical reaction between the target molecule (e.g., an antigen) and its chemical partner (e.g., antibody) that has been immobilized on the particle's surface. While the sample fluid dissolves the salt-sugar matrix, it also dissolves the particles and in one combined transport action the sample and conjugate mix while flowing through the porous structure. In this way, the analyte binds to the particles while migrating further through the third capillary bed. This material has one or more areas (often called stripes) where a third molecule has been immobilized by the manufacturer. By the time the sample-conjugate mix reaches these strips, analyte has been bound on the particle and the third 'capture' molecule binds the complex. After a while, when more and more fluid has passed the stripes, particles accumulate and the stripe-area changes color. Typically, there are at least two stripes: one (the control) that captures any particle and thereby shows that reaction conditions and technology worked fine, the second contains a specific capture molecule and only captures those particles onto which an analyte molecule has been immobilized. After passing these reaction zones the fluid enters the final porous material that acts as a waste container. Lateral Flow Tests can operate as either competitive or sandwich assays. In some embodiments, the lateral flow assay based on comparison of line intensity, line color, or a combination thereof to one or more reference lines. In some embodiments, the assay device comprises an optical device disposed for imaging or analyzing the lateral flow assay, and optionally a display device for visualizing the image of the lateral flow assay.

Non-limiting examples of bioluminescent (or chemiluminescent) assays performed fully or in part by a test module **108** is configured to perform one or more of an optical device for detecting bioluminescence, and a display device for visualizing the detected bioluminescence. In some embodiments, the at least one assay is a bioluminescence assay and wherein the assay device comprises an optical device for detecting bioluminescence, and a display device for visualizing the detected bioluminescence. In some embodiment, the bioluminescence assay is based on luciferase, which is a generic term for the class of oxidative enzymes used in bioluminescence and is distinct from a photoprotein. In these examples, typically, a bioluminescence assay comprises an imager and a detector.

In biological research, luciferase is commonly used as a reporter to assess the transcriptional activity in cells that are transfected with a genetic construct containing the luciferase gene under the control of a promoter of interest. Additionally, proluminescent molecules that are converted to luciferin upon activity of a particular enzyme can be used to detect enzyme activity in coupled or two-step luciferase assays. Such substrates have been used to detect caspase activity and cytochrome P450 activity, among others.

Luciferase can also be used to detect the level of cellular ATP in cell viability assays or for kinase activity assays. Luciferase can act as an ATP sensor protein through biotinylation. Biotinylation will immobilize luciferase on the cell-surface by binding to a streptavidin-biotin complex. This allows luciferase to detect the efflux of ATP from the cell and will effectively display the real-time release of ATP through bioluminescence. Luciferase can additionally be made more sensitive for ATP detection by increasing the luminescence intensity through genetic modification.

One example of bioluminescence ATP assay is the ATP Bioluminescence Assay Kit CLS II by Roche Applied Science, which is specially developed for applications in which constant light signals are required for kinetic studies of enzymes and metabolic studies, or if coupled enzymatic assays are applied. If ATP determinations are manually started, the CLS Kit provides high reproducibility due to the constant signal generation. However, the sensitivity of the kit is lower by a factor of 10 as compared to the ATP Bioluminescence Assay Kit HS II, which is recommended for determinations in the high-sensitivity range. The ATP Bioluminescence Assay Kit HS II also contains an efficient cell lysis reagent, and can be used for the detection of ATP in microorganisms or animal cells. The ATP Bioluminescence Assay Kit CLS II has a Detection limit of 10-11 M ATP (10-15 moles), using a luminometer.

In some embodiments, a test module **108** is configured to perform a cell cytometry assay, and wherein the assay device comprises a laser diode, a microcomputer, and an optical sensor. In some embodiments, at least one assay is a lateral flow assay based on comparison of line intensity, line color, or a combination thereof to one or more reference lines. In some embodiments, the lateral flow assay is selected from pregnancy assays or drug screening assays. In certain embodiments, the assay device comprises an optical device disposed for imaging the lateral flow assay, and a display device for visualizing the image of the lateral flow assay. Cell cytometry is a laser-based, biophysical technology employed in cell counting, cell sorting, biomarker detection and protein engineering, by suspending cells in a stream of fluid and passing them by an electronic detection apparatus. It allows simultaneous multiparametric analysis of the physical and chemical characteristics of up to thousands of particles per second. Flow cytometry is routinely used in the diagnosis of health disorders, especially blood cancers, but has many other applications in basic research, clinical practice and clinical trials. A common variation is to physically sort particles based on their properties, so as to purify populations of interest.

As a non-limiting example, a beam of light (usually laser light) of a single wavelength is directed onto a hydrodynamically focused stream of liquid. A number of detectors are aimed at the point where the stream passes through the light beam: one in line with the light beam (Forward Scatter or FSC) and several perpendicular to it (Side Scatter or SSC) and one or more fluorescence detectors. Each suspended particle from 0.2 to 150 micrometers passing through the beam scatters the ray, and fluorescent chemicals found in the particle or attached to the particle may be excited into emitting light at a longer wavelength than the light source. This combination of scattered and fluorescent light is picked up by the detectors, and, by analyzing fluctuations in brightness at each detector (one for each fluorescent emission peak), it is then possible to derive various types of information about the physical and chemical structure of each individual particle. FSC correlates with the cell volume and SSC depends on the inner complexity of the particle (i.e., shape of the nucleus, the amount and type of cytoplasmic granules or the membrane roughness). This is because the light is scattered off of the internal components of the cell. Some flow cytometers on the market have eliminated the need for fluorescence and use only light scatter for measurement. Other flow cytometers form images of each cell's fluorescence, scattered light, and transmitted light.

Fluorescence-activated cell sorting (FACS) is a specialized type of flow cytometry. It provides a method for sorting a heterogeneous mixture of biological cells into two or more containers, one cell at a time, based upon the specific light scattering and fluorescent characteristics of each cell. It is a useful scientific instrument, as it provides fast, objective and quantitative recording of fluorescent signals from individual cells as well as physical separation of cells of particular interest.

As a non-limiting example, the cell suspension is entrained in the center of a narrow, rapidly flowing stream of liquid. The flow is arranged so that there is a large separation between cells relative to their diameter. A vibrating mechanism causes the stream of cells to break into individual droplets. The device is adjusted so that there is a low probability of more than one cell per droplet. Just before the stream breaks into droplets, the flow passes through a fluorescence measuring station where the fluorescent character of interest of each cell is measured. An electrical charging ring is placed just at the point where the stream breaks into droplets. A charge is placed on the ring based on the immediately prior fluorescence intensity measurement, and the opposite charge is trapped on the droplet as it breaks from the stream. The charged droplets then fall through an electrostatic deflection device that diverts droplets into containers based upon their charge. In some devices, the charge is applied directly to the stream, and the droplet breaking off retains charge of the same sign as the stream. The stream is then returned to neutral after the droplet breaks off.

In addition to the ability to label and identify individual cells via fluorescent antibodies, cell cytometry can be used to measure cellular products such as cytokines, proteins, and other factors. Similar to ELISA sandwich assays, CBA assays use multiple bead populations typically differentiated by size and different levels of fluorescence intensity to distinguish multiple analytes in a single assay. The amount of the analyte captured is detected via a biotinylated antibody against a secondary epitope of the protein, followed by a streptavidin-R-phycoerythrin treatment. The fluorescent intensity of R-phycoerythrin on the beads is quantified on a flow cytometer equipped with a 488 nm excitation source. Concentrations of a protein of interest in the samples can be obtained by comparing the fluorescent signals to those of a standard curve generated from a serial dilution of a known concentration of the analyte.

In some embodiments, a test module **108** is configured to fully or partially perform DNA sequencing. In some embodiments, assays are based on DNA sequencing chips and wherein the test module **108** comprises an interface to the DNA sequencing chip.

In some embodiments, a test module **108** is configured to fully or partially perform an environmental assay selected from air quality assays, asbestos assays, water quality assays, soil content assays, or radon gas assays. In some embodiments, the at least one assay is an environmental assay and the assay device comprises gas chromatography (GC).

In some embodiments, a test module **108** is configured to detect radiation from a source.

In some embodiments, a device module **106** comprises one or more of the following functional devices: battery, a wireless data transmission device, a wired data transmission device, a microprocessor, a transitory memory device, a non-transitory memory device, an interface for receiving and recording signals from at least one vital sign detector, a luminescence recorder, a display device, a portable computing device, a data storage device, an optical device, a motor, a tag, an RF tag, a sensor, a thermal sensor, a speed sensor, a light sensor, an invisible light sensor, an electromagnetic wave sensor, an acoustic wave sensor, a transponder, a light source, an invisible light source, an electromagnetic wave source, a mechanical wave source, an acoustic wave source, a camera or other imager, a pump, an actuator, and combinations thereof. A camera or other imager as described herein may be precisely adjusted and controlled using software

In some embodiments, at least one device module **106** comprises a battery, and wherein the assay module further comprising a power inlet for receiving electric power from the battery.

In some embodiments, at least one device module **106** comprises an interface for at least one vital sign detector operatively associated with the functional module. In some embodiments, the at least one vital sign detectors collects body temperature, heart rate, blood pressure, respiratory rate, or combinations thereof. In some embodiments, the at least one functional module comprises a luminescence recorder adapted to record luminescence generated by the assay device. In some embodiments, the luminescence recorder is selected from a camera or other imager, a fluorescent light recorder, a UV recorder, a diode/amplifier type receiver, or combinations thereof. In some embodiments, the luminescence recorder is a built-in camera of a portable computing device.

In some embodiments, at least one device module **106** comprises a display **114** adapted to display test module **108** derived data, device module **106** derived data, sample related data, or combinations thereof. In some embodiments the display **114** is a high-resolution display. In some embodiments, the high-resolution display **114** further comprises a touch screen overlay. In some embodiments, the display is a display window of a portable computing device (not shown).

In some embodiments, at least one device module **106** comprises data storage to store information received from the assay module, the functional module, or a combination thereof.

It should be also be understood that one or more device modules **106** and/or a test modules **108** are suitable for use with embodiments of the device **102** that integrate with a computing device such as a smartphone. In these embodiments, a housing of a device **102** is configured to operably couple with a smartphone as well as one or more device modules **106** and/or test modules **108** that couple with the device **102** through one or more ports. In some embodiments, the portable computing device is coupled to at least one functional module. In some embodiments, the portable computing device includes a software module configured to communicate data to a user, wherein the data is acquired by the software on the computing device from a device module **106** and/or test module **108** coupled with the housing of the device **102**. In some embodiments, the data is securely communicated to the user through a server. In some embodiments, the data is an Internet server or a local access network server. In some embodiments, the user is a patient, a doctor, a nurse, or a medical practitioner.

### Systems

A system as described herein, in some embodiments, includes a device **102** (or any embodiment of said device) along with a third-party module **104,** wherein the third-party module **104** comprises any module that was not originally manufactured with device **102** and utilizes one or more universal interoperability coupler mechanisms to couple with the device **102**.

A system as described herein, in some embodiments, comprises a device **102** integrated with a computing device such as a smartphone. As previously described, in these embodiments, a housing of a device **102** is configured to operably couple with a smartphone as well as one or more device modules **106** and/or test modules **108** that couple with the device **102** through one or more ports.

A system as described herein, in some embodiments, comprises one or more test modules 108 configured to receive and analyze a sample or receive data from a container or third party module **104** that contains a sample. In these embodiments, the test module **108** is configured to transmit data through a wired or wireless transmission to a network, remote server, or computing device.

In some embodiments, a system includes connectivity to the Cloud or a remote server, wherein data is aggregated and, in some embodiments, analyzed further as described herein.

### Platforms

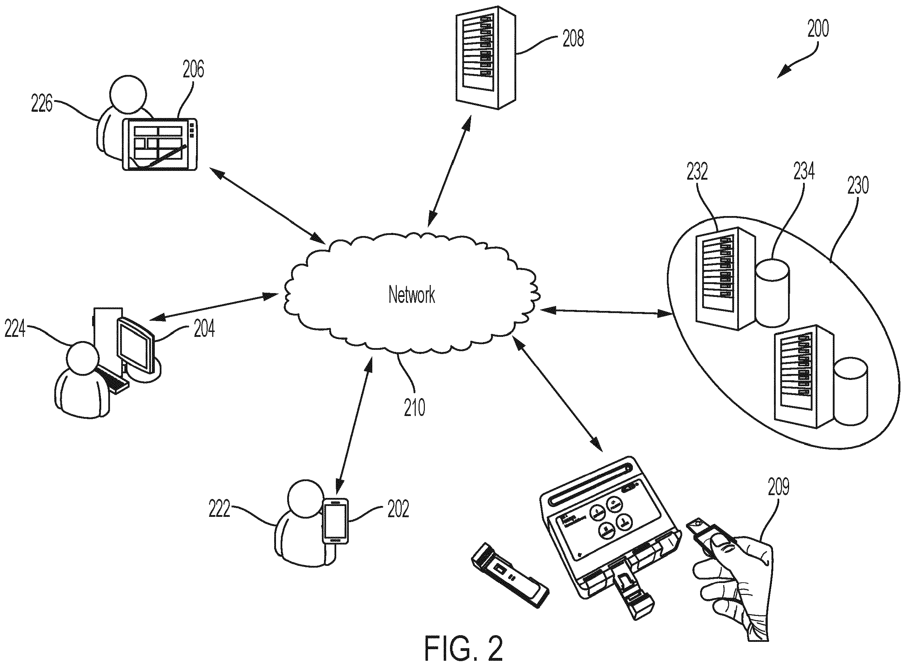

**FIG. 2** shows an example of environment that can be employed to execute implementations of one or more embodiments of the platform **200**. The example platform **200** includes computing devices **202**, **204**, **206**, **208**, modular hand-held point of care device or system **209**, a back-end system **230**, and a network **210**. In some embodiments, there are possibilities of connecting Peer-to-peer. For example, in some embodiments, there is a satellite link that communicates with the device using a device module. The satellite link may or may not connect to the internet, so this would be Peer-to-Peer

In some embodiments, the network **210** includes a local area network (LAN), wide area network (WAN), the Internet, or a combination thereof, and connects web sites, devices (e.g., the computing devices **202**, **204**, **206**, **208** and the medical device or system **209**) and back-end systems (e.g., the back-end system **230**). In some embodiments, the network **210** can be accessed over a wired and/or a wireless communications link. For example, mobile computing devices (e.g., the smartphone device **202** and the tablet device **206**), can use a cellular network to access the network **210**. In some embodiments, the users **222-226** includes physicians, patients, network technicians including network administrators and authorized programmers, nurses, residents, hospital administrators, insurers, and any other healthcare provider.

In the depicted example, the back-end system **230** includes at least one server system **232** and a data store **234**. In some embodiments, the at least one server system **232** hosts one or more computer-implemented services and portals employed within the described platform, such as described in **FIG. 2**, that users **222-226** can interact with using the respective computing devices 102-106. For example, the computing devices **202-206** may be used by respective users **222-226** to generate and retrieve reports regarding patient scans taken by the medical device or system **209** through services hosted by the back-end system **230** (see **FIG**. **2**). In some embodiments, the back-end system **230** provides an API service with which the server computing device **208** may communicate.

In some embodiments, back-end system **230** includes server-class hardware type devices. In some embodiments, back-end system **230** includes computer systems using clustered computers and components to act as a single pool of seamless resources when accessed through the network **210**. For example, such embodiments may be used in data center, cloud computing, storage area network (SAN), and network attached storage (NAS) applications. In some embodiments, back-end system **230** is deployed using a virtual machine(s).

In some embodiments, the computing devices **202**, **204**, **206** include any appropriate type of computing device, such as a desktop computer, a laptop computer, a handheld computer, a tablet computer, a personal digital assistant (PDA), a cellular telephone, a network appliance, a camera or other imager, a smart phone, an enhanced general packet radio service (EGPRS) mobile phone, a media player, a navigation device, an email device, a game console, or an appropriate combination of any two or more of these devices or other data processing devices. In the depicted example, the computing device **202** is a smartphone, the computing device **204** is a desktop computing device, and the computing device **206** is a tablet-computing device. In some embodiments, the server computing device **208** includes any appropriate type of computing device, such as described above for computing devices **202-206** as well as computing devices with server-class hardware. In some embodiments, the server computing device **208** includes computer systems using clustered computers and components to act as a single pool of seamless resources. It is contemplated, however, that implementations of the present disclosure can be realized with any of the appropriate computing devices, such as those mentioned previously.

In some embodiments, data that is received from one or more devices and/or test modules as described herein is aggregated and wirelessly transmitted to or received from a remote server (e.g. the Cloud) where data from multiple sources where it is stored. For example, data from multiple devices and/or test modules may be aggregated for the purpose of quality control or performance monitoring. For example, data from multiple devices and/or test modules may be aggregated for the purpose of patient study. For example, data from a device and/or test module relating to one or more samples from a patient may be aggregated with other patient data including physiologic data (e.g. from a monitoring device) and/or patient medical record data.

### Multiplex Testing

Any of the devices, systems, and platforms described herein may be used in multiplex testing wherein one or more test modules produce either multiple different assays and/or assay multiple samples in a synchronized manner.

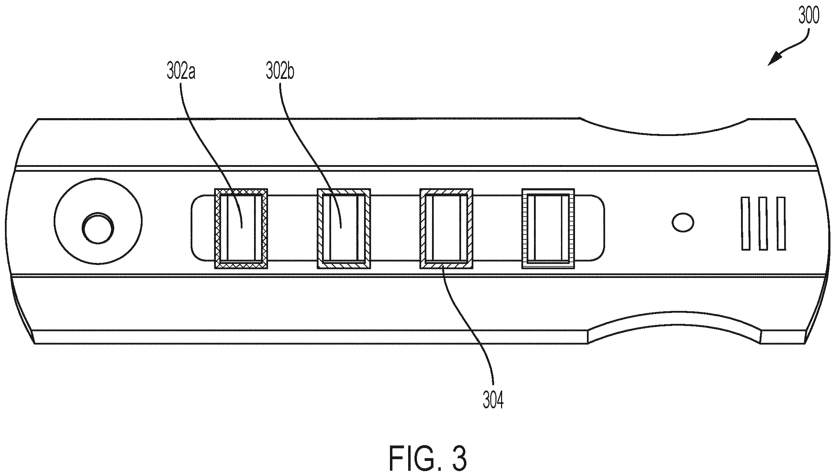

**FIG. 3** shows an exemplary embodiment of a test module **300** as described herein configured as a lateral flow cartridge. In some embodiments, a camera or other optical reader or imager is used to determine a test line **304**. In some embodiments, a camera is configured to allow the operator to fine turn the position of the entire aperture, or move the edges of the aperture to get a more perfect fit compared to the position found mathematically by the device. In some embodiments a result comprises identification of a presence or absence of a test line. For example, in the case of drugs of abuse, the lack of a test line usually means a maximum amount of target. For example, in the case of immuno detection, it would mean that there is no target in the sample.

Generally, in these embodiments, multiple samples **302a** and **302b** may be assayed on a single cartridge **300**. Determining a test line **304** allows for each of the samples to be discretely assayed on the single cartridge in a multiplex fashion.

In some embodiments, two or more test modules as described herein are combined to perform two or more assays on a single sample.

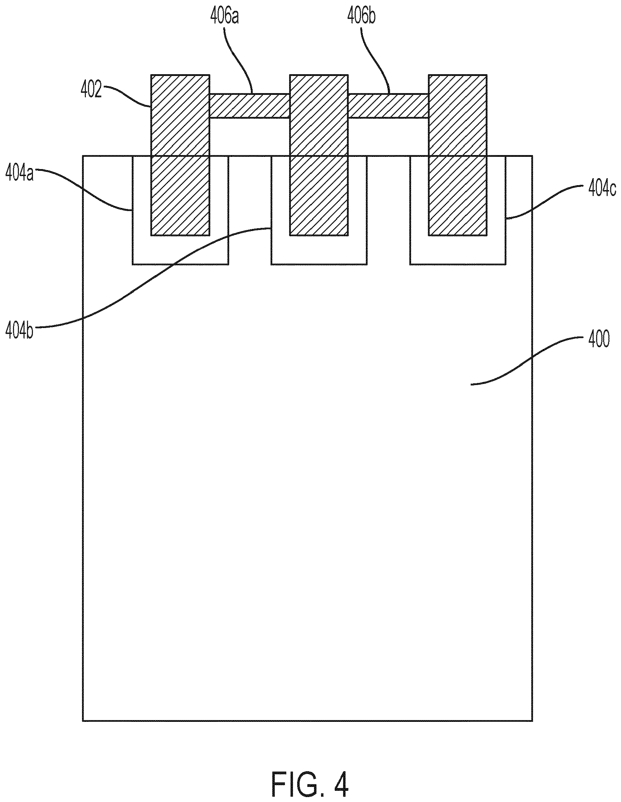

**FIG. 4** is a schematic representation of a device **400** as described herein coupled with three test devices that are coupled to one another to form a multiplex testing device **402.** As shown, connecting channels **406a**, **406b** connect the three testing devices of multiplex testing device **402** and position them so that each testing device will be received by a separate port **402a**, **402b**, and **404c**. In some embodiments, the three test modules each is configured to run a different assay and they are run simultaneously or essentially simultaneously in order to collect different types of information from a biologic sample. In some embodiments, at least two of the test modules of a multiplex test module **402** are the same type of assay. In some embodiments, at least one test module of the multiplex test module **402** is configured to receive a different type of sample than the other test modules of the multiplex test module **402**. In some embodiments instead of channels **406a** and **406b**, the individual test modules are connected by solid non-communicating connections. It should be understood that while in the example of **FIG. 4** a multiplex module comprising of three test modules was shown, other embodiments are suitable for use with the devices, systems, and platforms described herein. In some embodiments, a multiplex module comprises 20 test modules. In some embodiments, a multiplex module comprises 15 test modules. In some embodiments, a multiplex module comprises 10 test modules. In some embodiments, a multiplex module comprises 9 test modules. In some embodiments, a multiplex module comprises 8 test modules. In some embodiments, a multiplex module comprises 7 test modules. In some embodiments, a multiplex module comprises 6 test modules. In some embodiments, a multiplex module comprises 5 test modules. In some embodiments, a multiplex module comprises 4 test modules. In some embodiments, a multiplex module comprises 2 test modules.

Described herein is a hand-held point of care monitoring device, system, and platform configured to carry out tests and measurements including, but not limited to, biologic sample testing and physical parameter (e.g. vital sign) measurement.

As used herein the phrase "universal interoperability coupler" means a mechanism and/or a means for a device as described herein to operate with multiple different types of modules. A universal interoperability coupler enables a device as described herein to operate with multiple different types of modules by enabling the device to transmit and/or receive data from different types of modules and/or communicate with different types of modules. Non-limiting types of modules that a device as described herein operates with, using a universal interoperability coupler, include modules having varying dimensions (i.e. length, width, and height) and/or varying input types, ports, prongs, or connectors used to make hardwired connections, and/or varying types of means for transmitting data, and/or modules manufactured by different manufacturers.. A universal interoperability coupler includes mechanical means for adjusting a dimension of a port that receives modules having varying dimensions and/or means for drawing data from modules having varying types of hardwire connectors or wireless transmission means (including, for example, RF, optical, WiFi, cellular, Bluetooth, and/or ultrasound).

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention.

## Claims

1. A point of care testing device, comprising:
(a) a test module (108) configured to receive a container that contains a biologic sample, and perform an assay on the biologic sample to generate an assay result;
(b) a processor configured to receive data from the test module (108);
(c) a transmitter configured to transmit data from the test module (108) to a mobile computing device or a remote server; and
(d) a universal interoperability coupler configured to operably couple the test module (108) with the container,
**characterized in that** the universal interoperability coupler comprises one or more internal adjustment bars with external handles that allow for the user to adjust the external handles to positions that allow the container to fit inside of the test module (108).

2. The device of claim 1, wherein the test module (108) includes a camera, an imaging modality, or a detection device is configured to take an image or detect the biologic sample.

3. The device of claim 2, wherein the processor includes software configured to cause the processor to evaluate the image.

4. The device of any preceding claim, wherein the test module (108) is configured for multiplex testing by receiving at least two biologic samples at once that are separated within the test module (108).

5. The device of claim 4, wherein the at least two biologic samples are positioned on a test strip.

6. The device of claim 4 or 5, when depending on claim 2, wherein the camera, imaging modalities, or detection devices is configured to determine a physical boundary of each of the at least two samples that are separated.

7. The device of claim 1, further comprising two or more cameras or imaging modalities, wherein images formed by the two or more cameras or imaging modalities are assembled by the processor to create a single image with a wider field of view.

8. The device of claim 1, further comprising a camera, wherein the camera comprises a lighting source, and a receiver configured to receive a fluorescent signal.

9. The device of any preceding claim, wherein the transmitter is configured to operate on one or more transmission technologies selected from 3G communication protocols, 4G communication protocols, GSM standards, CDMA protocols, IEEE 802.11 standards, Bluetooth protocols, satellite communications, visible light communications, infrared communications, a hardwired connection, and near field communications.

10. The device of any preceding claim, comprising a non-transitory computer readable medium including software configured to provide communication protocols that allow communication with other computing devices through one of a Blue Tooth connection, a WiFi connection, an RF connection.

11. The device of any preceding claim, wherein the device is configured to operate with containers each manufactured by a different manufacturer.

12. The device of any preceding claim, further comprising a shroud or shield configured to shield the device from ambient noise.

13. The device of any preceding claim, wherein the device is configured to receive at least two containers each having a different shape than the other.

14. The device of any preceding claim, comprising a luminescence recorder adapted to record luminescence associated with the biologic sample.

15. The device of any preceding claim, comprising a plurality of said test modules (108), each test module (108) configured to
(i) receive a biologic sample or a container that contains a biologic sample,
(ii) perform a assay on the biologic sample, and
(iii) generate a result of the assay.

16. The device of claim 15, further comprising a portable frame with a plurality of ports (110), wherein at least two ports (110) are configured to simultaneously receive test modules (108).

## Patentansprüche

1. Point-of-Care-Testvorrichtung, die Folgendes umfasst:
(a) ein Testmodul (108), das dafür konfiguriert ist, einen Behälter aufzunehmen, der eine biologische Probe enthält, und einen Test an der biologischen Probe durchzuführen, um ein Testergebnis zu erzeugen;
(b) einen Prozessor, der dafür konfiguriert ist, Daten von dem Testmodul (108) zu empfangen;
(c) einen Sender, der dafür konfiguriert ist, Daten von dem Testmodul (108) an eine mobile Computervorrichtung oder einen Remote-Server zu übertragen; und
(d) einen universellen Interoperabilitätskoppler, der dafür konfiguriert ist, das Testmodul (108) mit dem Behälter funktionsfähig zu koppeln, **dadurch gekennzeichnet, dass** der universelle Interoperabilitätskoppler eine oder mehrere interne Einstellstangen mit externen Griffen umfasst, die es dem Benutzer ermöglichen, die externen Griffe in Positionen zu bringen, die es ermöglichen, dass der Behälter in das Testmodul (108) passt.

2. Vorrichtung nach Anspruch 1, wobei das Testmodul (108) eine Kamera, eine Bildgebungsmodalität oder eine Erfassungsvorrichtung enthält, die dafür konfiguriert ist, ein Bild aufzunehmen oder die biologische Probe zu erfassen.

3. Vorrichtung nach Anspruch 2, wobei der Prozessor eine Software enthält, die dafür konfiguriert ist, den Prozessor zu veranlassen, das Bild auszuwerten.

4. Vorrichtung nach einem der vorhergehenden Ansprüche, wobei das Testmodul (108) für Multiplex-Tests konfiguriert ist, indem mindestens zwei biologische Proben gleichzeitig empfangen werden, die innerhalb des Testmoduls (108) getrennt werden.

5. Vorrichtung nach Anspruch 4, wobei die mindestens zwei biologischen Proben auf einem Teststreifen positioniert sind.

6. Vorrichtung nach Anspruch 4 oder 5, wenn abhängig von Anspruch 2, wobei die Kamera, die Bildgebungsmodalitäten oder die Erfassungsvorrichtungen dafür konfiguriert sind, eine physische Grenze jeder der mindestens zwei Proben zu bestimmen, die voneinander getrennt sind.

7. Vorrichtung nach Anspruch 1, die ferner zwei oder mehr Kameras oder Bildgebungsmodalitäten umfasst, wobei die von den zwei oder mehr Kameras oder Bildgebungsmodalitäten erzeugten Bilder von dem Prozessor zusammengesetzt werden, um ein einzelnes Bild mit einem breiteren Sichtfeld zu erzeugen.

8. Vorrichtung nach Anspruch 1, die ferner eine Kamera umfasst, wobei die Kamera eine Lichtquelle und einen Empfänger umfasst, der dafür konfiguriert ist, ein Fluoreszenzsignal zu empfangen.

9. Vorrichtung nach einem der vorhergehenden Ansprüche, wobei der Sender dafür konfiguriert ist, mit einer oder mehreren Übertragungstechnologien zu arbeiten, die aus 3G-Kommunikationsprotokollen, 4G-Kommunikationsprotokollen, GSM-Standards, CDMA-Protokollen, IEEE 802.11-Standards, Bluetooth-Protokollen, Satellitenkommunikation, Kommunikation mit sichtbarem Licht, Infrarotkommunikation, einer festverdrahteten Verbindung und Nahfeldkommunikation ausgewählt sind.

10. Vorrichtung nach einem der vorhergehenden Ansprüche, die ein nicht-flüchtiges computerlesbares Medium umfasst, das Software enthält, die dafür konfiguriert ist, Kommunikationsprotokolle bereitzustellen, die die Kommunikation mit anderen Computervorrichtungen über eine Bluetooth-Verbindung, eine WiFi-Verbindung oder eine RF-Verbindung ermöglichen.

11. Vorrichtung nach einem der vorhergehenden Ansprüche, wobei die Vorrichtung dafür konfiguriert ist, mit Containern zu arbeiten, die jeweils von einem anderen Hersteller hergestellt wurden.

12. Vorrichtung nach einem der vorhergehenden Ansprüche, die ferner eine Verkleidung oder Abschirmung umfasst, die dafür konfiguriert ist, die Vorrichtung vor Umgebungsgeräuschen abzuschirmen.

13. Vorrichtung nach einem der vorhergehenden Ansprüche, wobei die Vorrichtung dafür konfiguriert ist, mindestens zwei Behälter aufzunehmen, die jeweils eine andere Form als die anderen haben.

14. Vorrichtung nach einem der vorhergehenden Ansprüche, die einen Lumineszenzrekorder umfasst, der dafür ausgelegt ist, Lumineszenz aufzuzeichnen, die der biologischen Probe zugeordnet ist.

15. Vorrichtung nach einem der vorhergehenden Ansprüche, die eine Vielzahl der genannten Testmodule (108) umfasst, wobei jedes Testmodul (108) für Folgendes konfiguriert ist:
(i) Empfangen einer biologischen Probe oder eines Behälters, der eine biologische Probe enthält,
(ii) Durchführen eines Tests an der biologischen Probe und
(iii) Erzeugen eines Ergebnisses des Tests.

16. Vorrichtung nach Anspruch 15, die ferner einen tragbaren Rahmen mit einer Vielzahl von Anschlüssen (110) umfasst, wobei mindestens zwei Anschlüsse (110) dafür konfiguriert sind, gleichzeitig Testmodule (108) aufzunehmen.

## Revendications

1. Dispositif de test au point de service, comprenant :
(a) un module de test (108) configuré pour recevoir un contenant qui contient un échantillon biologique, et effectuer une analyse sur l'échantillon biologique afin de générer un résultat d'analyse ;
(b) un processeur configuré pour recevoir des données à partir du module de test (108) ;
(c) un émetteur configuré pour émettre des données depuis le module de test (108) vers un dispositif informatique mobile ou un serveur à distance ; et
(d) un coupleur à interopérabilité universelle configuré pour coupler de manière fonctionnelle le module de test (108) au contenant,
**caractérisé en ce que** le coupleur à interopérabilité universelle comprend une ou plusieurs barres d'ajustement internes avec des poignées externes qui permettent à l'utilisateur d'ajuster les poignées externes à des positions qui permettent au contenant de s'adapter à l'intérieur du module de test (108).

2. Dispositif de la revendication 1, dans lequel le module de test (108) inclut une caméra, une modalité d'imagerie, ou un dispositif de détection qui est configuré pour prendre une image ou détecter l'échantillon biologique.

3. Dispositif de la revendication 2, dans lequel le processeur inclut un logiciel configuré pour amener le processeur à évaluer l'image.

4. Dispositif de n'importe quelle revendication précédente, dans lequel le module de test (108) est configuré pour effectuer des tests en multiplex grâce à la réception d'au moins deux échantillons biologiques à la fois qui sont séparés au sein du module de test (108).

5. Dispositif de la revendication 4, dans lequel les au moins deux échantillons biologiques sont positionnés sur une bandelette de test.

6. Dispositif de la revendication 4 ou 5, lorsqu'elle est dépendante de la revendication 2, dans lequel la caméra, les modalités d'imagerie, ou les dispositifs de détection sont configuré/es pour déterminer une limite physique de chacun des au moins deux échantillons qui sont séparés.

7. Dispositif de la revendication 1, comprenant en outre deux ou plusieurs caméras ou modalités d'imagerie, dans lequel les images formées par les deux ou plusieurs caméras ou modalités d'imagerie sont assemblées par le processeur afin de créer une image unique avec un champ de vision plus large.

8. Dispositif de la revendication 1, comprenant en outre une caméra, dans lequel la caméra comprend une source d'éclairage, et un récepteur configuré pour recevoir un signal fluorescent.

9. Dispositif de n'importe quelle revendication précédente, dans lequel l'émetteur est configuré pour fonctionner sur une ou plusieurs technologies de transmission sélectionnées parmi : protocoles de communication 3G, protocoles de communication 4G, normes GSM, protocoles CDMA, normes IEEE 802.11, protocoles Bluetooth, communications satellitaires, communications par lumière visible, communications par infrarouges, une connexion câblée, et communications en champ proche.

10. Dispositif de n'importe quelle revendication précédente, comprenant un support non transitoire lisible par ordinateur incluant un logiciel configuré pour fournir des protocoles de communication qui permettent des communications avec d'autres dispositifs informatiques par l'intermédiaire d'une connexion parmi une connexion Blue Tooth, une connexion WiFi, une connexion RF.

11. Dispositif de n'importe quelle revendication précédente, dans lequel le dispositif est configuré pour fonctionner avec des contenants dont chacun est fabriqué par un fabricant différent.

12. Dispositif de n'importe quelle revendication précédente, comprenant en outre un couvercle ou un écran configuré pour protéger le dispositif contre le bruit ambiant.

13. Dispositif de n'importe quelle revendication précédente, le dispositif étant configuré pour recevoir au moins deux contenants dont chacun a une forme différente de l'autre.

14. Dispositif de n'importe quelle revendication précédente, comprenant un enregistreur de luminescence conçu pour enregistrer la luminescence associée à l'échantillon biologique.

15. Dispositif de n'importe quelle revendication précédente, comprenant une pluralité desdits modules de test (108), chaque module de test (108) étant configuré pour
(i) recevoir un échantillon biologique ou un contenant qui contient un échantillon biologique,
(ii) effectuer une analyse sur l'échantillon biologique, et
(iii) générer un résultat de l'analyse.

16. Dispositif de la revendication 15, comprenant en outre un cadre portatif avec une pluralité de ports (110), dans lequel au moins deux ports (110) sont configurés pour recevoir simultanément des modules de test (108).
